# EUROPEAN PATENT APPLICATION

(11) **EP 3 960 132 A1**
(43) Date of publication of application: **02.03.2022**
(21) Application number: 19926456.5
(22) Date of filing: 26.04.2019
(51) Int. Cl.: A61F 5/10

(54) **HALLUX VALGUS CORRECTION SUPPORTER**

(71) Applicant: Sakamoto, Kiyoshi, Tsuyama-shi Okayama 708-0824 (JP)
(72) Inventor: Sakamoto, Kiyoshi, Tsuyama-shi Okayama 708-0824 (JP)
(74) Representative: De Arpe Tejero, Manuel
(86) International application number: PCT/JP2019/017973
(87) International publication number: WO 2020/217464

(57) **Abstract**

A hallux valgus orthopedic supporter which enables comfortable wear for a long time is disclosed. In the hallux valgus orthopedic supporter, in which a supporting member 10 is configured to be worn around a tarsus by applying a supporting member body 11 to vicinity of a heel, placing each of a first protruding part 12 and a second protruding part 13 from left-and-right side of an instep to upper side of the instep with the second protruding part 13 overlapping an outer surface of the first protruding part 12 and fastening a second touch fastener 15 to a first touch fastener 14, and a big-toe sheath 20 is configured to be pulled backward by attaching a rear end of a traction band 30 to medial side of the supporting member body 11, a tip-side edge area of the first protruding part 12 and a tip-side edge area of the first touch fastener 14 have substantially the same arc-like shape and are overlaid.

## Description

### TECHNICAL FIELD

The present disclosure relates to a hallux valgus orthopedic supporter for correcting hallux valgus, in which a big toe (first toe) is bent toward a long toe (second toe).

### BACKGROUND

The present inventor has already proposed a hallux valgus orthopedic supporter for correcting hallux valgus disclosed in PTL1.

The hallux valgus orthopedic supporter (referred to as "orthotics" in PTL1) disclosed in PTL1 includes a supporter 11 illustrated in Fig. 1 of PTL1, a big-toe sheath 12 and traction bands 13, 14, 15 illustrated in Fig. 2 of PTL1. The supporter 11 is a member for being worn around a tarsus as illustrated in Fig. 3 and Fig. 4 of PTL1. The big-toe sheath 12 is a member for being worn on a big toe. The traction bands 13, 14, 15 have their front ends fixed to the big-toe sheath 12 and extend backward from medial side of the big-toe sheath 12.

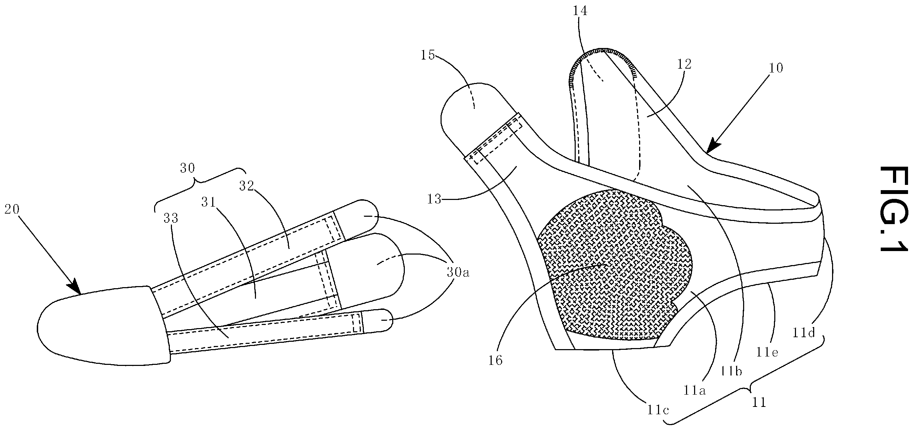

Among the members included in the hallux valgus orthopedic supporter disclosed in PTL1, the supporter 11 includes, as illustrated in Fig. 1 of PTL1, a supporter body 16 (referred to as "main part" in PTL1) for covering vicinity of a heel, a first protruding part 17 and a second protruding part 18 (referred to as "left wing part" and "right wing part" in PTL1, respectively) each protruding from left-and-right side of the supporter body 16 so that each of the first protruding part 17 and the second protruding part 18 is configured to cover an upper surface of a instep, a first touch fastener 21 provided on the first protruding part 17, and a second touch fastener 22 provided at a tip end of the second protruding part 18.

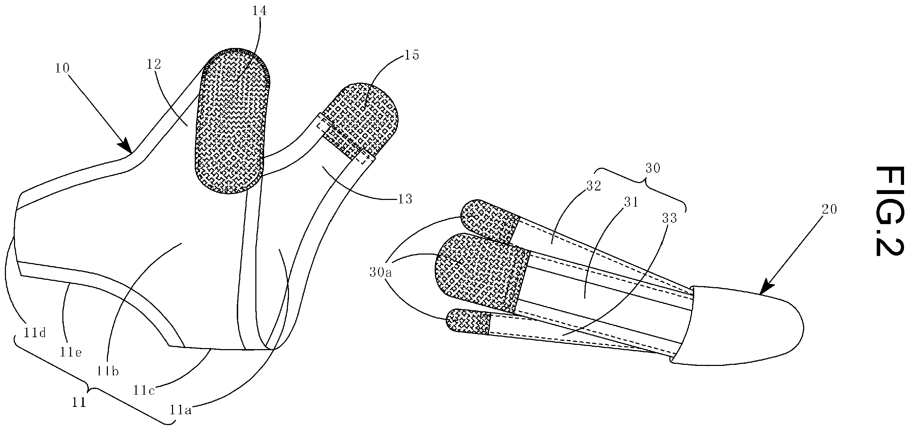

The hallux valgus orthopedic supporter disclosed in PTL1 is designed to be used in the following manner. Place each of the first protruding part 17 and the second protruding part 18 from left-and-right side of the instep to upper side of the instep with the second protruding part 18 overlapping an outer surface of the first protruding part 17. Fasten the second touch fastener 22 to the first touch fastener 21, so that the supporter 11 is worn around the tarsus. Attach rear ends (touch fasteners 26, 27, 28 illustrated in Fig. 2 of PTL1) of the traction bands 13, 14, 15 to medial side (a touch fastener 25 illustrated in Fig. 2 of PTL1) of the supporter body 16.

Consequently, the big-toe sheath 12 is pulled backward, and an external force (tension of traction bands 13, 14, 15) toward the medial side is applied to the tip of the big toe. Thus, continuous wearing of the hallux valgus orthopedic supporter disclosed in PTL1 gradually corrects hallux valgus. It is concerned, however, that desirable corrective effect may not be achieved if the big-toe sheath 12 rotates upon wearing the hallux valgus orthopedic supporter disclosed in PTL1. In this regard, it is also disclosed in PTL1 that rotation of the big-toe sheath 12 is preventable by using three traction bands 13, 14, 15, namely a main traction band 13 in the center and subsidiary traction bands 14, 15 arranged above and below the main traction band 13.

### Citation List

### Patent Literature

PTL1: Japanese Laid-Open Patent Publication No. 2005-152218

### SUMMARY

### Technical Problems

Although the hallux valgus orthopedic supporter disclosed in PTL1 is effective in correcting hallux valgus over a long period of time, there is a concern that the wearer may feel pain on the instep because of the following reasons.

As described above, when using the hallux valgus orthopedic supporter disclosed in PTL1, the supporter 11 is worn around the tarsus by overlapping the second protruding part 18 on the upper (outer) surface of the first protruding part 17 and fastening the second touch fastener 22 to the first touch fastener 21. When the supporter 11 is worn, the first protruding part 17 and the second protruding part 18 tighten the instep of the foot. The lower positioned first protruding part 17 has, as illustrated in Fig. 1 of PTL1, two substantially right-angled corners at a tip-side edge area thereof. When the supporter 11 is worn, the above-mentioned corners of the first protruding part 17 are likely to bite into the instep of the wearer.

The supporter 11 is usually made entirely of soft stretchable fabric, including the first protruding part 17. The corners of the stretchable fabric itself are less likely to cause pain to the wearer. However, as the above-mentioned first touch fastener 21 is provided on the outer surface of the first protruding part 17 overlaying thereon, the first protruding part 17 is more rigid (stiffer) than other parts in the supporter 11. The above-mentioned corners at the tip-side edge area of the first protruding part 17 thus likely to cause the wearer feel pain as the corners poke against the instep.

In view of the aforementioned problems, the present disclosure discloses a hallux valgus orthopedic supporter which has part(s) placed on an upper surface side of a instep (e.g. the first protruding part described above) less likely to bite into the instep, thereby enabling comfortable wear with less pain for a long time.

### Disclosed Solution

A hallux valgus orthopedic supporter, comprising:
a supporting member configured to be worn around a tarsus;
a big-toe sheath configured to be worn on a big toe; and
a traction band configured to extend backward from medial side of the big-toe sheath,
the supporting member including
a supporting member body for covering vicinity of a heel,
a first protruding part and a second protruding part each protruding from left-and-right side of the supporting member body so that each of the first protruding part and the second protruding part is configured to cover an upper surface of an instep,
a first touch fastener provided on the first protruding part, and
a second touch fastener provided at a tip end of the second protruding part,
wherein the supporting member is configured to be worn around the tarsus by applying the supporting member body to the vicinity of the heel, placing each of the first protruding part and the second protruding part from left-and-right side of the instep to upper side of the instep with the second protruding part overlapping an outer surface of the first protruding part and fastening the second touch fastener to the first touch fastener, and the big-toe sheath is configured to be pulled backward by attaching a rear end of the traction band to medial side of the supporting member body,
wherein a tip-side edge area of the first protruding part and a tip-side edge area of the first touch fastener have substantially the same arc-like shape and are overlaid.

The "medial side" means a side of the wearer's foot which faces center of the wearer's body in left-right direction. "Medial side of the big toe", for example, means the left side of the big toe (left side as seen by the wearer of the hallux valgus orthopedic supporter) if the big toe is on the right foot, and means the right side of the big toe (right side as seen by the wearer of the hallux valgus orthopedic supporter) if the big toe is on the left foot. In this context, a side of the wearer's foot which faces outside of the wearer's body in left-right direction is sometimes referred to as "lateral side" hereinafter.

The "arc-like shape" means smooth, rounded curve (curve without corners), and is not limited to strict circular arc. Therefore, elliptical curves, quadratic curves and the like are also included in the "arc-like shape".

In the present disclosure, the tip-side edge area of the first protruding part and the tip-side edge area of the first touch fastener have substantially the same arc-like shape and are overlaid. The tip of the first protruding part and the first touch fastener is thus less likely to poke against the instep when the supporting member is worn around the tarsus. It enables the wearer to comfortably wear the hallux valgus orthopedic supporter with less pain for a long time.

Finish of the tip-side edge areas of the first protruding part and the first touch fastener are both not particularly limited. In one or more embodiments, the tip-side edge area of the first protruding part and the tip-side edge area of the first touch fastener are sewn together by whipstitch or overcast stitch. As a result, the edge of the fabric forming the first protruding part is finished (processed to prevent fraying) without being folded back at the tip-side of the first protruding part, making the tip-side edge areas of the first protruding part thinner. The tip-side edge area of the first protruding part is thus less likely to apply pressure to the instep of the wearer. In addition, the threads wrapped around the tip-side edge area of the first protruding part and the tip-side edge area of the first touch fastener cover a ridgeline between a tip end surface and a fastening surface of the first touch fastener, as well as a ridgeline between the tip end surface and a back surface (the side opposite the fastening surface) of the first touch fastener, making those ridgelines less likely to contact directly with the instep. The ridgelines of the first touch fastener are thus less likely to poke against the instep. Consequently, an advantage is achieved in that the wearer even less likely to feel pain on the instep.

In some embodiments, the entire area of the second touch fastener overlaps the second protruding part. In this case, the entire area of the supporting member where the second touch fastener is installed is likely to be thicker. It is thus concerned that the instep of the wearer is likely to be compressed. In this regard, in one or more embodiments, only a base-side edge area of the second touch fastener overlaps the second protruding part, and the other area(s) (area(s) other than the base-side edge area) of the second touch fastener does not overlap the second protruding part.

In at least one embodiment, the base-side edge area of the second touch fastener is sewn to a tip-side edge area of the second protruding part so that a tip-side portion of the second touch fastener protrudes from the tip-side edge area of the second protruding part. Most area of the second touch fastener, except for the base-side edge area, is thus does not overlap the second protruding part, making the area of the supporting member where the second touch fastener is installed thinner.

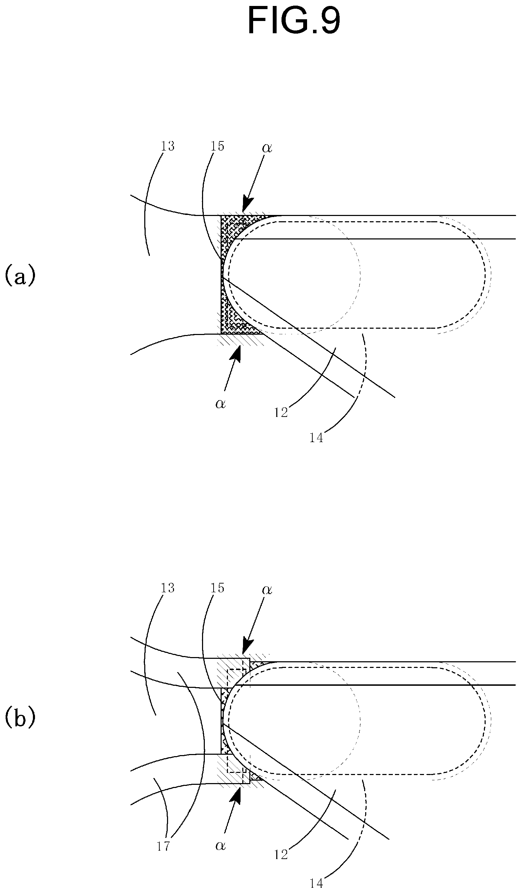

However, even if the area of the supporting member where the second touch fastener is installed made thinner as described above, it is still concerned that pain may occur on the instep for the following reasons. In the present disclosure, as described above, the tip-side edge areas of the first protruding part and the first touch fastener have substantially the same arc-like shape and are overlaid. When the second touch fastener 15 is fastened close to the tip end of the first touch fastener 14 as illustrated in Fig. 9 (a) (e.g., when the instep circumference is large), it is concerned that area(s) α (indicated by the shaded hatching) on the base-side of the second touch fastener 15 is exposed from the both sides of the tip-side edge areas of the first protruding part 12 and the first touch fastener 14, and corners of the area(s) α pokes against the instep. It is also concerned that the second touch fastener 15 in the area(s) α touches the instep, causing discomfort to the wearer.

In this regard, in one or more embodiments, the second protruding part 13 has piping 17 on both lateral edges, the piping 17 extending to the point where the piping 17 overlaps a base end of the second touch fastener 15 as illustrated in Fig. 9 (b). The area(s) α and/or the corners thereof is/are thus less likely to directly touch the instep of the wearer. Consequently, an advantage is achieved in that pain on the instep is less likely to occur.

### Advantageous Effect

The hallux valgus orthopedic supporter of the present disclosure has part(s) placed over the upper surface of instep (e.g., the first protruding part described above) less likely to bite into instep, thereby enabling comfortable wear with less pain for a long time.

### Brief Description of Drawings

Fig. 1 illustrates a hallux valgus orthopedic supporter for right foot viewed from side;
Fig. 2 illustrates the hallux valgus orthopedic supporter for right foot viewed from the opposite side of Fig. 1;

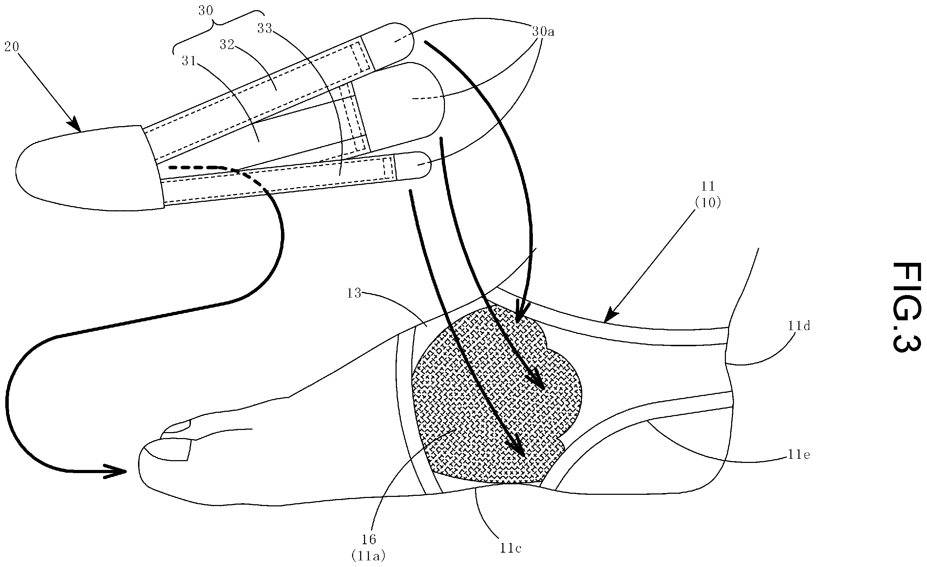

Fig. 3 illustrates the hallux valgus orthopedic supporter for right foot in the process of being worn on a right foot viewed from side, illustrating the state in which a supporting member has been worn;

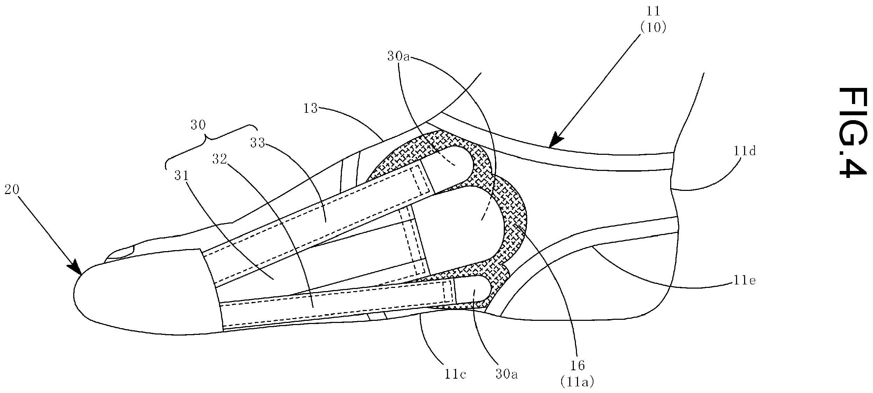

Fig. 4 illustrates the hallux valgus orthopedic supporter for right foot which has been worn on the right foot viewed from side;

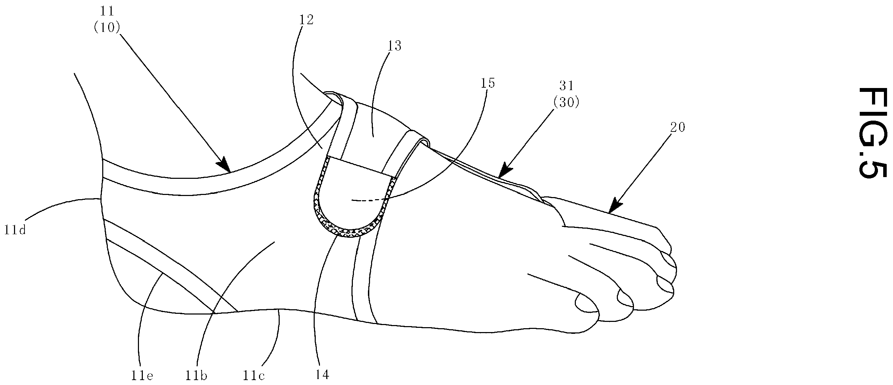

Fig. 5 illustrates the hallux valgus orthopedic supporter for right foot which has been worn on the right foot viewed from the opposite side of Fig. 4;

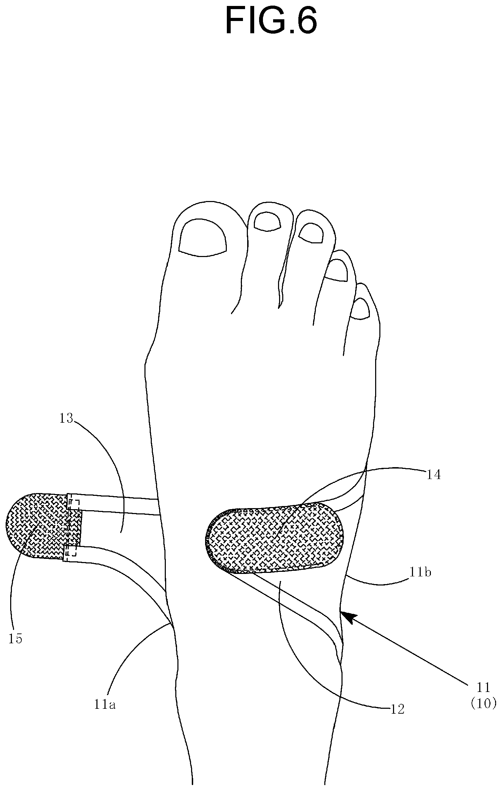

Fig. 6 illustrates the hallux valgus orthopedic supporter for right foot in the process of being worn on the right foot viewed from top, illustrating the state in which a first protruding part of the supporting member is placed over an instep;

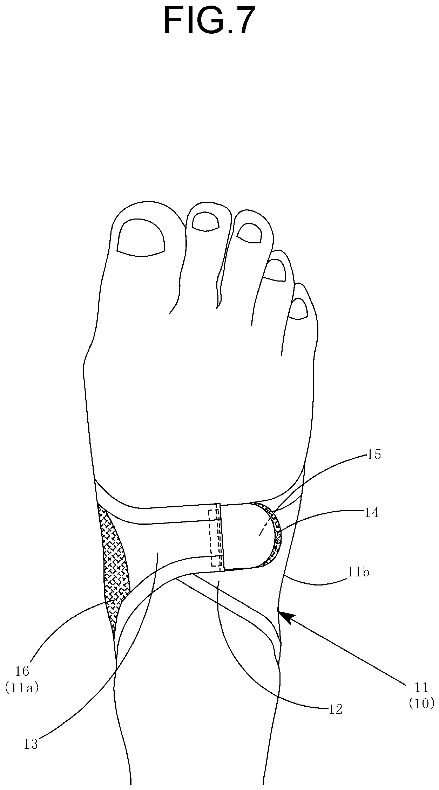

Fig. 7 illustrates the hallux valgus orthopedic supporter for right foot in the process of being worn on the right foot viewed from top, illustrating the state in which a second protruding part is overlapped with an outer surface of the first protruding part of the supporting member;

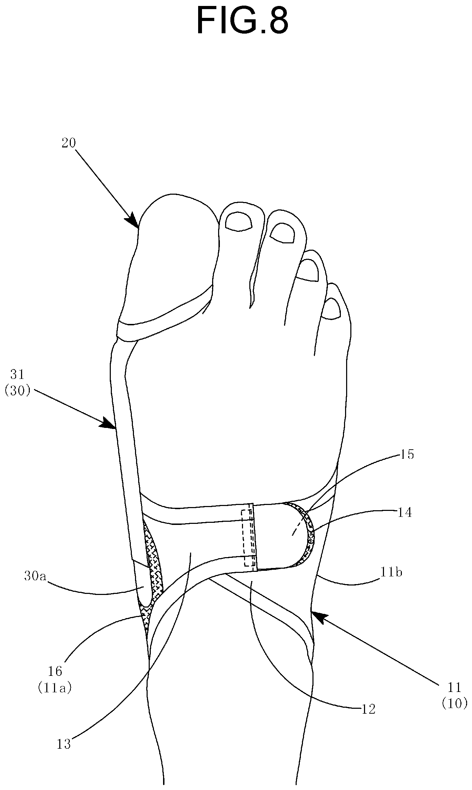

Fig. 8 illustrates the hallux valgus orthopedic supporter for right foot which has been worn on the right foot viewed from top; and
Fig. 9 illustrates a second touch fastener on the second protruding part is fastened to a first touch fastener on the first protruding part in the hallux valgus orthopedic supporter for right foot, viewed from fastening surface side of the second touch fastener.

### EMBODIMENTS

Specific modes of embodiments according to the present disclosure are described below, referring to the accompanying drawings. A hallux valgus orthopedic supporter can be either for right foot, to be worn on a right foot, or for left foot, to be worn on a left foot. In the following, for convenience of explanation, the hallux valgus orthopedic supporter for right foot is mainly described. Descriptions of the hallux valgus orthopedic supporter for left foot is omitted when possible. In some embodiments, the hallux valgus orthopedic supporter for left foot has a substantially symmetrical structure with the hallux valgus orthopedic supporter for right foot. Configuration(s) described below for the hallux valgus orthopedic supporter for right foot thus may be applied to the hallux valgus orthopedic supporter for left foot by switching the left-and-right side.

Fig. 1 illustrates the hallux valgus orthopedic supporter for right foot viewed from side (medial side). Fig. 2 illustrates the hallux valgus orthopedic supporter for right foot viewed from the opposite side of Fig. 1 (lateral side). In one or more embodiments, the hallux valgus orthopedic comprises a supporting member 10, a big-toe sheath 20 and a traction band 30 as illustrated in Fig. 1 and Fig. 2.

Fig. 3 illustrates the hallux valgus orthopedic supporter for right foot in the process of being worn on a right foot viewed from side (medial side), illustrating the state in which the supporting member 10 has been worn, and the big-toe sheath 20 is about to be worn. The supporting member 10 is a member for being worn around a tarsus as illustrated in Fig. 3. Fig. 4 and Fig. 5 illustrate the hallux valgus orthopedic supporter for right foot which has been worn on the right foot. Fig. 4 illustrates a view from the medial side, and Fig. 5 illustrates a view from the lateral side. The big-toe sheath 20 is a member to be worn on a big toe as illustrated in Fig. 4 and Fig. 5. The traction band 30, as illustrated in Fig. 1, has a front end fixed to the big-toe sheath 20, and extends backward from the medial side of the big-toe sheath 20.

The supporting member 10 literally functions as a "supporter" to moderately tighten the tarsus of the wearer, as well as a member to hold a rear end of the traction band 30. The supporting member 10 has a supporting member body 11, a first protruding part 12 and a second protruding part 13 as illustrated in Fig. 1. Materials to form the supporting member 10 is not limited. In some embodiments, the supporting member 10 is made of stretchable fabric. Consequently, wearing comfort of the supporting member 10 is enhanced. In one or more embodiments, the supporting member 10 is made of a warp knitted fabric woven with nylon fibers and polyester fibers so that the warp knitted fabric stretches in both wale and course directions. In at least one embodiment, the warp knitted fabric is a Torisukin (registered trademark).

Thickness of the fabric (stretchable fabric) forming the supporting member 10 is not limited. It is concerned, however, that necessary strength for the supporting member 10 is less likely to be secured if the fabric is too thin. In this regard, in some embodiments, the thickness of the fabric forming the supporting member 10 is 0.1 mm or more. In one or more embodiments, the thickness of the fabric forming the supporting member 10 is 0.3 mm or more. On the other hand, it is also concerned that the wearing comfort of the supporting member 10 is less likely to be enhanced, for example by making the wearer feel discomfort in the tarsus when wearing the supporting member 10, if the fabric forming the supporting member 10 is too thick. In this regard, in some embodiments, the thickness of the fabric forming the supporting member 10 is 1 mm or less. In one or more embodiments, the thickness of the fabric forming the supporting member 10 is 0.7 mm or less. In at least one embodiment, the thickness of the fabric forming the supporting member 10 is approximately 0.5 mm. When this kind of thin fabric is used, however, it is concerned that even a slight sewing error is likely to cause wrinkles, and defective products as a result.

The supporting member body 11 is a part for covering vicinity of a heel of the wearer. In some embodiments, the supporting member body 11 includes a medial surface area 11a for covering vicinity of a medial malleolus as illustrated in Fig. 3, a lateral surface area 11b for covering vicinity of a lateral malleolus as illustrated in Fig. 5, a lower connection area 11c connecting lower edge of the medial surface area 11a and lower edge of the lateral surface area 11b for covering a lower surface of an arch of foot, and a rear connection area 11d connecting rear edge of the medial surface area 11a and rear edge of the lateral surface area 11b for covering a rear surface of the tarsus. The supporting member body 11 also includes a heel insertion hole 11e for inserting the heel between the lower connection area 11c and the rear connection area 11d. By inserting the heel into the heel insertion hole 11e upon wearing the supporting member 10 around the tarsus, the supporting member 10 worn around the tarsus is more likely to be stabilized.

The first protruding part 12 is provided protruding from upper side of the lateral surface area 11b of the supporting member body 11 as illustrated in Fig. 2. The first protruding part 12 is designed to be placed over an upper surface of an instep to cover thereof as illustrated in Fig. 6. The second protruding part 13 is provided protruding from upper side of the medial surface area 11a of the supporting member body 11 as illustrated in Fig. 2. The second protruding part 13 is designed to be placed overlapping an outer surface (a surface which faces away from the instep when the hallux valgus orthopedic supporter is worn. The same applies hereinafter.) of the first protruding part 12 as illustrated in Fig. 7. Fig. 6 and Fig. 7 illustrate the hallux valgus orthopedic supporter for right foot in the process of being worn on the right foot viewed from top. Fig. 6 illustrates the state in which the first protruding part 12 of the supporting member 10 is placed over the instep. Fig. 7 illustrates the state in which the second protruding part 13 is overlapped with the outer surface of the first protruding part 12 of the supporting member 10.

A first touch fastener 14 is provided on the outer surface of the first protruding part 12 as illustrated in Fig. 2. A tip-side edge area of the first protruding part 12 and a tip-side edge area of the first touch fastener 14 have substantially the same arc-like shape and are overlaid. In some embodiments, the first touch fastener 14 is formed in a strip, wherein both edge area in the longitudinal direction formed in arc-like curvy shapes. One of those arc-like edge areas of the first touch fastener 14 is overlapped with the arch-formed tip-side edge area of the first protruding part 12, so that substantially the entire first touch fastener 14 overlaps the outer surface of the first protruding part 12. By sewing the first touch fastener 14 along a periphery thereof to the first protruding part 12, the first touch fastener 14 is fixed to the first protruding part 12. A tip-side rim of the first touch fastener 14 is overlaid to a tip-side rim of the first protruding part 12. The tip-side edge area of the first protruding part 12 and the tip-side edge area of the first touch fastener 14 are sewn together by whipstitch or overcast stitch. By adopting whipstitch or overcast stitch here, an advantage is achieved in that, even in the embodiments wherein the supporting member 10 is formed with thin stretchable fabric as described above, the stretchable fabric is likely to be neatly finished with less wrinkles.

A second touch fastener 15 is provided at a tip end of the second protruding part 13. In some embodiments, a base-side edge area of the second touch fastener 15 is sewn to a tip-side edge area of the second protruding part 13 so that nearly the entire area of the second touch fastener 15 protrudes from the tip-side edge area of the second protruding part 13. In other words, nearly the entire area of the second touch fastener 15 does not overlap the second protruding part 13. In one or more embodiments, the second touch fastener 15 is formed in a strip, wherein one edge area in the longitudinal direction formed in arc-like curvy shape. The other edge area (an edge area on the side that is not formed in arc-like shape) of the second touch fastener 15 is overlapped with the tip-side edge area of the second protruding part 13, making only the other edge area (the base-side edge area) of the second touch fastener 15 overlaps the second protruding part 13. By sewing the second touch fastener 15 along the base-side edge area thereof to the tip-side edge area of the second protruding part 13, the second touch fastener 15 is fixed to the second protruding part 13. The second touch fastener 15 is sewn to the second protruding part 13 without tip-side edge area of the second protruding part 13 being folded back. A fastening surface of the second touch fastener 15 is arranged to face inner side (side which faces the instep when the hallux valgus orthopedic supporter is worn. The same applies hereinafter.). This second touch fastener 15 is a component for being fastened to the first touch fastener 14.

In some embodiments, the supporting member 10 is designed to be worn around the tarsus as illustrated in Fig. 7, with the first protruding part 12 and the second protruding part 13 fixed to each other by inserting the heel into the heel insertion hole 11e on the supporting member 10, placing the first protruding part 12 over the instep as illustrated in Fig. 6 and fastening the second touch fastener 15 at a tip end of the second protruding part 13 to the first touch fastener 14 on the first protruding part 12. The second protruding part 13 is typically pulled and extended (stretched than a natural length thereof) when the second touch fastener 15 is fastened to the first touch fastener 14. Consequently, the tarsus of the wearer is moderately tightened by the supporting member 10, enabling the supporting member 10 to firmly support the tarsus. The first touch fastener 14 is formed in a strip which extends in the left-right direction when placed on the tarsus. The tightening force of the supporting member 10 is thus able to be adjusted by adjusting the position (left and right fastening positions) of the second touch fastener 15 to the first touch fastener 14.

As described above, it is concerned that the wearer of the hallux valgus orthopedic supporter may feel pain on the instep if the edge area of the first touch fastener 14 pokes against the instep of the wearer when the second touch fastener 15 is fastened to the first touch fastener 14. In this regard, in some embodiments, the tip-side edge area of the first touch fastener 14 formed in an arc-like shape, and is overlaid with the tip-side edge area of the first protruding part 12 formed similarly in an arc-like shape as described above. In addition, the tip-side edge area of the first protruding part 12 and the tip-side edge area of the first touch fastener 14 are sewn together by whipstitch or overcast stitch. Consequently, the tip-side edge areas of the first protruding part 12 and the first touch fastener 14 have smooth shape with substantially no corners, and the tip-side edge areas of the first protruding part 12 is finished to be thin without substantially being folded back. Furthermore, a ridgeline between a fastening surface and a tip end surface of the first touch fastener 14, as well as a ridgeline between a back surface (the side opposite the fastening surface) and the tip end surface of the first touch fastener 14 is covered with threads, making those ridgelines less likely to contact directly with the instep. An advantage is thus achieved in that the tip-side edge areas of the first protruding part 12 and the first touch fastener 14 are less likely to poke against the instep, making the wearer less likely to feel pain even when wearing the hallux valgus orthopedic supporter for a long time.

However, in the embodiments wherein the tip-side edge areas of the first protruding part 12 and the first touch fastener 14 have substantially the same arc-like shape and are overlaid, it is concerned that, when the second touch fastener 15 is fastened close to the tip end of the first touch fastener 14 as illustrated in Fig. 9 (a) (e.g., when the instep circumference is large), area(s) α (indicated by the shaded hatching) on base-side of the second touch fastener 15 is exposed from the both sides of the tip-side edge areas of the first protruding part 12, and corners of the area(s) α pokes against the instep. It is also concerned that the second touch fastener 15 in the area(s) α touches the instep, and the wearer may feel discomfort.

In this regard, in one or more embodiments, the second protruding part 13 has piping 17 on both lateral edges, the piping 17 extending to the point where the piping 17 overlap a base end of the second touch fastener 15 as illustrated in Fig. 9 (b). Fig. 9 illustrates the second touch fastener 15 on the second protruding part 13 is fastened to the first touch fastener 14 on the first protruding part 12 in the supporting member, viewed from the fastening surface side of the second touch fastener 15. Fig. 9 (a) illustrates an embodiment not having piping on the second touch fastener 15, while Fig. 9 (b) illustrates an embodiment having piping 17 on the second touch fastener 15. The area(s) α and/or the corners thereof is/are thus less likely to directly touch the instep of the wearer by the piping 17 extending to the point where the piping 17 overlaps the base end of the second touch fastener 15. In addition, by having the piping 17, prevention of fraying at edge of the fabric forming the supporting member 10, which is an intrinsic purpose of the piping 17, is achieved without having additional component.

It is also concerned that, if a fastened area of the first touch fastener 14 and the second touch fastener 15 is too thick when the two are fastened each other, the fastened area is likely to be pressed against the instep by a sock and/or a shoe worn over the supporting member 10, and may cause pain on an area of the instep of the wearer where the fastened area overlaps. In this regard, in some embodiments, nearly the entire area of the second touch fastener 15 does not overlap the fabric forming the second protruding part 13 as described above, making the fastened area above less likely to be thick.

It is further concerned that, as the first protruding part 12 where the first touch fastener 14 is provided is formed with a relatively thin fabric, pain may occur on the instep if a base-side edge area (an area on the opposite side of the tip-side) of the first touch fastener 14 pokes against the instep of the wearer through the stretchable fabric underneath (the stretchable fabric forming the first protrusion part 12). In this regard, in some embodiments, not only the tip-side edge area of the first touch fastener 14 but also the base-side edge area thereof is formed in an arc-like shape. The base-side edge area of the first touch fastener 14 is thus less likely to poke against the instep of the wearer.

The supporting member 10 has the first touch fastener 14 and the second touch fastener 15 for wearing the supporting member 10 around the tarsus as described above. In some embodiments, the supporting member 10 also has a third touch fastener 16 in addition to those two fasteners as illustrated in Fig. 2. The third touch fastener 16 is fixed (sewn in one or more embodiments) to an outer surface of the medial surface area 11a of the supporting member 10. The third touch fastener 16 is a component for being fastened a fourth touch fastener 30a (Fig. 1) provided on the rear end of the traction band 30 described below.

Fig. 8 illustrates the hallux valgus orthopedic supporter for right foot which has been worn on the right foot viewed from top. The big-toe sheath 20 is design to be worn covering the big toe as illustrated in Fig. 8. The big-toe sheath 20 is therefore formed in a sheath-like, bag-like or cap-like shape so that the big toe is be able to be inserted therein. Materials to form the big-toe sheath 20 is not limited. In some embodiments, the big-toe sheath 20 is made of stretchable fabric, as is the case of the supporting member 10. Consequently, wearing comfort of the big-toe sheath 20 is enhanced. In one or more embodiments, the big-toe sheath 20 is made of substantially the same fabric as the supporting member 10 (the warp knitted fabric woven with nylon fibers and polyester fibers so that the warp knitted fabric stretches in both wale and course directions).

The traction band 30 is formed in a band-like shape. A front end of the traction band 30 is fixed (sewn in one or more embodiments) to the medial side of the big-toe sheath 20 as illustrated in Fig. 8. The traction band 30 is a member for pulling the big-toe sheath 20 backward. Materials to form the traction band 30 is not limited. In some embodiments, the traction band 30 is made of stretchable fabric, as is the case of the supporting member 10. Consequently, the big-toe sheath 20 is pulled backward elastically, making the big-toe sheath 20 less likely to be subjected to excessive force. In one or more embodiments, the traction band 30 is made of substantially the same fabric as the supporting member 10 (the warp knitted fabric woven with nylon fibers and polyester fibers so that the warp knitted fabric stretches in both wale and course directions).

The fourth touch fastener 30a is provided on the rear end of the traction band 30 as illustrated in Fig. 2. A fastening surface of the fourth touch fastener 30a is arranged to face inner side (side which faces the foot when the hallux valgus orthopedic supporter is worn. The same applies hereinafter.). The fourth touch fastener 30a is a component for being fastened to the third touch fastener 16 on the supporting member body 11 as illustrated in Fig. 4 and Fig. 8. The fourth touch fastener 30a is fastened to the third touch fastener 16 so that the traction band 30 is extended (stretched than a natural length thereof). Consequently, a retracting force of the traction band 30 works as a traction force for pulling the big-toe sheath 20 backward.

Thus, by attaching the rear end of the traction band 30 to the supporting member body 10, and pulling the medial side of the big-toe sheath 20 backward with the traction band 30, tip of the big toe wearing the big-toe sheath 20 is pulled toward the medial side. As a result, hallux valgus is likely to be corrected. The third touch fastener 16, which is for being fastened the rear end (the fourth touch fastener 30a) of the traction band 30, is provided in a relatively wide area of the supporting member body 11 across the front-to-back direction as illustrated in Fig. 3. As a result, the traction force of the traction band 30 for pulling the big-toe sheath 20 is adjustable by adjusting fastening position (position in the front-to-back direction) of the fourth touch fastener 30a against the third touch fastener 16.

In some embodiments, only one traction band 30 is provided for each big-toe sheath 20. In the case of only one traction band 30, however, it is concerned that the big-toe sheath 20 is likely to rotate around the big toe, and the traction band 30 is likely to be twisted, making the big-toe sheath 20 likely to be pulled in the twisted direction by the traction band 30 during long wearing of the hallux valgus orthopedic supporter. It is concerned that desirable corrective effect is less likely to be achieved when the big-toe sheath 20 is thus pulled in the twisted direction. In this regard, in one or more embodiments, a plurality of the traction band 30 is provided. As a result, an advantage is achieved in that the big-toe sheath 20 is less likely to rotate around the big toe, and the problem above is less likely to occur.

In one or more embodiments, the plurality of the traction band 30 comprises three bands, namely a main traction band 31 for pulling middle part of a medial side rear edge of the big-toe sheath 20, an upper subsidiary traction band 32 for pulling upper part of the medial side rear edge of the big-toe sheath 20, and a lower subsidiary traction band 33 for pulling lower part of the medial side rear edge of the big-toe sheath 20 as illustrated in FIG. 4. The medial side rear edge of the big-toe sheath 20 is thus pulled at a multiple position across the top-to-bottom direction with the plurality of the traction band 30, making the big-toe sheath 20 less likely to rotate around the big toe. The similar advantage is also achievable by one wide traction band 30. However, in one or more embodiments comprising the plurality of the traction band 30, additional advantage is achieved in that the traction force is adjustable for each traction band 30, such as weakening the traction force by the upper subsidiary traction band 32 and strengthening the traction force by the lower subsidiary traction band 33.

Widths of the main traction band 31, the upper subsidiary traction band 32 and the lower subsidiary traction band 33 are either not particularly limited. In many embodiments, the width of the main traction band 31 is wider than that of the upper subsidiary traction band 32 or the lower subsidiary traction band 33. In some embodiments, the width of the main traction band 31 is wider than (approximately twice as wide as) that of the upper subsidiary traction band 32 and the lower subsidiary traction band 33, with the main traction band 31 arranged inside (closer to the skin of the foot), and the upper subsidiary traction band 32 and the lower subsidiary traction band 33 overlapped outside of the main traction band 31. This makes it easy to attach the rear end of the traction band 30, for example, in steps including attaching a rear end of the main traction band 31 to the third touch fastener 16 of the supporting member 10 and then attaching rear ends of the upper subsidiary traction band 32 and the lower subsidiary traction band 33 to the third touch fastener 16 of the supporting member 10.

In one or more embodiment, the third touch fastener 16, which is for being fastened the fourth touch fastener 30a provided on the rear end of the traction band 30, is provided in a relatively wide area of the supporting member body 11 across the top-to-bottom direction. The rear ends of the three bands (the main traction band 31, the upper subsidiary traction band 32 and the lower subsidiary traction band 33) of the traction band 30 are thus simultaneously attachable to the third touch fastener 16, with each of their positions adjustable in the top-to-bottom direction to some extent. As a result, direction of the traction force of the traction band 30 for pulling the big-toe sheath 20 is finely adjustable.

As described above, the hallux valgus orthopedic supporter of the present disclosure is designed to be worn by wearing the supporting member 10 around the tarsus near the heel, wearing the big-toe sheath 20 on the big toe and attaching the rear end of the traction band 30 to the medial side of the supporting member 10. The backward traction force applied to the medial side of the big-toe sheath 20 by the traction band 30 pulls the tip of the big toe toward the medial side, making it likely that hallux valgus is corrected. The pain on the instep of the wearer (the pain caused when the tip ends of the first protruding part and/or the first touch fastener 14 poke against the instep), which is concerned with the hallux valgus orthopedic supporter disclosed in PTL1, is less likely to occur with the hallux valgus orthopedic supporter of the present disclosure, enabling comfortable wear of the hallux valgus orthopedic supporter for a long time.

### Reference Signs List

- 10: Supporting member
- 11: Supporting member body
- 11a: Medial surface area
- 11b: Lateral surface area
- 11c: Lower connection area
- 11d: Rear connection area
- 11e: Heel insertion hole
- 12: First protruding part
- 13: Second protruding part
- 14: First touch fastener
- 15: Second touch fastener
- 16: Third touch fastener
- 17: Piping
- 20: Big-toe sheath
- 30: Traction band
- 30a: Fourth touch fastener
- 31: Main traction band
- 32: Upper subsidiary traction band
- 33: Lower subsidiary traction band

## Claims

1. A hallux valgus orthopedic supporter, comprising:
a supporting member configured to be worn around a tarsus;
a big-toe sheath configured to be worn on a big toe; and
a traction band configured to extend backward from medial side of the big-toe sheath,
the supporting member including
a supporting member body for covering vicinity of a heel,
a first protruding part and a second protruding part each protruding from left-and-right side of the supporting member body so that each of the first protruding part and the second protruding part is configured to cover an upper surface of an instep,
a first touch fastener provided on the first protruding part, and
a second touch fastener provided at a tip end of the second protruding part,
wherein the supporting member is configured to be worn around the tarsus by applying the supporting member body to the vicinity of the heel, placing each of the first protruding part and the second protruding part from left-and-right side of the instep to upper side of the instep with the second protruding part overlapping an outer surface of the first protruding part and fastening the second touch fastener to the first touch fastener, and the big-toe sheath is configured to be pulled backward by attaching a rear end of the traction band to medial side of the supporting member body,
wherein a tip-side edge area of the first protruding part and a tip-side edge area of the first touch fastener have substantially the same arc-like shape and are overlaid.

2. The hallux valgus orthopedic supporter according to claim 1, wherein the tip-side edge area of the first protruding part and the tip-side edge area of the first touch fastener are sewn together by whipstitch or overcast stitch.

3. The hallux valgus orthopedic supporter according to claim 1 or 2, wherein:
a base-side edge area of the second touch fastener is sewn to a tip-side edge area of the second protruding part so that a tip-side portion of the second touch fastener protrudes from the tip-side edge area of the second protruding part; and
the second protruding part has piping on both lateral edges, the piping extending to the point where the piping overlaps a base end of the second touch fastener.
